(19) Europäisches Patentamt
European Patent Office
Office européen des brevets

(11) Veröffentlichungsnummer: **0 165 492 B1**

(12) **EUROPÄISCHE PATENTSCHRIFT**

(45) Veröffentlichungstag der Patentschrift: **14.10.92**

(51) Int. Cl.⁵: **A61K 37/02**, C07K 5/00, C07K 7/00

(21) Anmeldenummer: **85106212.5**

(22) Anmeldetag: **21.05.85**

# (54) Verfahren zur Herstellung wundheilungsfördernder Präparationen und solche Präparationen.

(30) Priorität: **22.05.84 DD 263249**

(43) Veröffentlichungstag der Anmeldung: **27.12.85 Patentblatt 85/52**

(45) Bekanntmachung des Hinweises auf die Patenterteilung: **14.10.92 Patentblatt 92/42**

(84) Benannte Vertragsstaaten: **AT BE CH DE FR GB IT LI SE**

(56) Entgegenhaltungen:

| | |
|---|---|
| EP-A- 0 034 504 | EP-A- 0 047 647 |
| EP-A- 0 124 317 | EP-A- 0 133 225 |
| EP-A- 0 160 307 | DD-A- 222 782 |
| DD-A- 234 230 | DE-A- 2 945 239 |
| FR-A- 2 503 561 | GB-A- 2 163 166 |
| US-A- 3 862 114 | US-A- 4 353 823 |

**CHEMICAL ABSTRACTS, Band 90, 1979, Columbus, OH (US); Seite 634, Nr. 104363v&NUM;**

(73) Patentinhaber: **BERLIN-CHEMIE Aktiengesellschaft
Glienicker Weg 125
O-1199 Berlin(DE)**

(72) Erfinder: **Jentzsch, Klaus Dieter, Prof. Dr.
Frankenstrasse 32
O-1185 Berlin(DE)**
Erfinder: **Buntrock, Peter, Dr.
Bornholmer Strasse 19
O-1171 Berlin(DE)**
Erfinder: **Oehme, Peter, Prof. Dr.
Hubertusstrasse 43
O-1409 Mühlenbeck/Summt(DE)**
Erfinder: **Kohl, Andreas
Einbecker Strasse 101
O-1136 Berlin(DE)**
Erfinder: **Neubert, Klaus, Dr.
Block 281/04
O-4090 Halle-Neustadt(DE)**

(74) Vertreter: **Lehn, Werner, Dipl.-Ing. et al
Hoffmann, Eitle & Partner Patentanwälte Arabellastrasse 4
W-8000 München 81(DE)**

EP 0 165 492 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

### Anwendungsgebiet der Erfindung

Die Erfindung betrifft die in den Ansprüchen näher gekennzeichneten Verwendungen. Ein Verfahren zur Herstellung wundheilungsfördernder Präparationen, die das Peptid Lys-Pro und seine Derivate enthalten, wird im folgenden näher beschrieben. Solche Peptide regen die Bildung von Granulationsgewebe an und bereiten damit den Wundgrund für den anschließenden Epithelisierungsvorgang bei Haut- und Schleimhautwunden oder für die Eigenhauttransplantation vor.
Die Zubereitungen sind für die direkte oder indirekte lokale Applikation in, an und auf die Wunde geeignet. Durch die Erfindung wird es möglich, Zubereitungen verfügbar zu haben, die wirksam und mit hoher Verträglichkeit den Wundheilungsprozeß begünstigen und beschleunigen.
Die Erfindung ist zur Anwendung in der Medizin, Veterinärmedizin und Kosmetik geeignet.

### Charakteristik der bekannten technischen Lösungen

Bekannt ist eine Reihe pharmazeutischer Zubereitungen mit unterschiedlichen Wirkstoffen oder Wirkstoffkombinationen, die - in Wundnähe oder auf Wunden gebracht - eine entzündungshemmende (DE-OS 2945 239; US-PS 3965 260) oder keimhemmende bzw. -vernichtende (Desinfektionsmittel, Antibiotika, Sulfonamide) Wirkung besitzen (HAUSCHILD, F.: Pharmakologie und Grundlagen der Toxikologie, VEB Georg Thieme Verlag Leipzig, 1973; FORTH, W.: HENSCHLER, D.; RÜMMEL, W.; Allgemeine und spezielle Pharmakologie und Toxikologie, Bibliographisches Institut Mannheim, Wien, Zürich, B.I.-Wissenschaftsverlag, 1980). Bekannt ist ferner die Möglichkeit der Überdeckung von Wunden mit Klebepflastern (DE-OS 2165 549 und 2443 180; DD-WP 102 078), mit adsorbierenden Granulaten (z. B. Zelluloseregenerat) oder mit synthetischem Hautersatz (DE-OS 2708 822 und 2845 686), der wahlweise mit einem geeigneten Wirkstoff imprägniert sein kann (DD-WP 145 225). Alle aufgeführten Zubereitungen und die Wundabdekkungsmaterialien besitzen zwar einen günstigen Einfluß auf die Wundheilung, indem sie **entweder** in Abhängigkeit von Art und Quantität des Wirkstoffes Entzündungsprozesse hemmen, Kontaminationen mit Eitererregern vermindern, vereiteln oder bereits in die Wunde eingedrungene Erreger hemmen oder abtöten **oder** auf physikalische Weise (Abschirmung, Sekretaufnahme, Verhinderung der Austrocknung u. a.) den Heilungsprozeß günstig beeinflussen, aber in keinem Fall wird eine spezifische unmittelbare Wirkung auf den biologischen Grundprozeß der Wundheilung, nämlich die Ausbildung des den Wundgrund ausfüllenden Granulationsgewebes, entfaltet.

Bekannt ist allerdings die Stimulierung der Granulationsgewebsbildung durch einen Rohextrakt aus dem Gehirn von Schlachttieren, der "Fibroblast Growth Factor"-Aktivität enthält und zur Imprägnierung von Wundabdeckungsmaterialien geeignet ist (DD-WP 145 225). Dieser Hirnrohextrakt besitzt aber den Nachteil, daß er nicht nur den Wirkstoff, sondern zusätzlich Begleitproteine und andere Hirnaufbau- und funktionsstoffe enthält, deren Art im einzelnen unbekannt und deren Anteil von Herstellungscharge zu Herstellungscharge wechselnd ist, so daß eine ausreichende chemische Charakterisierung des Präparates nicht möglich ist. Selbst in stärker aufgereinigter Form sind zudem wegen der engen Beziehung zum Nervenmyelin bei wiederholter Anwendung allergische Nebenreaktionen zu erwarten (GOSPODAROWICZ, D.; Mol. Cell. Biochem. 25, 79, 1979).

Bekannt ist schließlich die entzündungshemmende und wundheilungsstimulierende Wirkung von L-Carnosin ($\beta$-Alanyl-L-Histidin) (NAGAI, K. et al: Arzneimittel-Forsch./Drug Res. 20, 1876, 1970; NAGAI K., T. JAMANE: Heterocycles 10, 277, 1978). Bei intraperitonealer Applikation ließen sich bei Versuchstieren (Ratten) experimentell gesetzte Wunden und bei lokaler Applikation (Salbe, Lutschtabletten) durch Zahnextraktion entstandene Wunden beim Menschen günstig beeinflussen. Hierzu sind jedoch außerordentlich hohe Dosen (20 mg/kg Ratte intraperitoneal) erforderlich. Diese Tatsache ist zusammen mit Befunden anderer Autoren (FITZPATRICK, D., H. FISHER: Fed. Proc. 40, 3/2, 2514, 1981), nach denen L-Carnosin keine eigentliche Wirkung besitzt, sondern lediglich ein Lieferant der Aminosäure Histidin und damit des die zelluläre Synthese bewirkenden Histamins ist, der Grund dafür, daß sich dieser Wirkstoff in der medizinischen Praxis nicht durchgesetzt hat.

### Ziel der Erfindung

Das Ziel der Erfindung besteht darin, Präparationen zur Verfügung zu stellen, die bei unmittelbarer oder mittelbarer Applikation ins Wundgebiet den Primärschritt des Heilungsprozesses von Wunden, nämlich die Bildung des Granulationsgewebes, anregen und beschleunigen, hinsichtlich des enthaltenen Wirkstoffes

eindeutig chemisch definiert sind, gut verträglich sind, wahlweise mit bekannten Wirkstoffen mit unterstützender biologischer Wirkung kombiniert werden können und pharmazeutisch geeignete Trägerstoffe zur Formulierung enthalten, wodurch eine Anwendung am Menschen möglich wird und über bisher bekannte Möglichkeiten hinaus die Wundheilung, vornehmlich die Bildung des Granulationsgewebes, günstig beeinflußt und dadurch der Wundheilungsvorgang insgesamt gefördert wird.

Darlegung des Wesens der Erfindung

Der Erfindung liegt die Aufgabe zugrunde, Verfahren zur Herstellung von Präparationen mit wundheilungsfördernder Wirksamkeit zu entwickeln, die den Grundprozeß der Wundheilung, nämlich die Bildung des Granulationsgewebes, anregen. Dazu werden biologisch wirksame Peptide, wahlweise bzw. zweckgebunden ergänzt durch bereits bekannte Wirkstoffe definierter Wirkungsqualität - wie z. B. Antibiotika oder Sulfonamide - unter Verwendung chemisch inerter, in der Pharmazie gebräuchlicher Lösungsmittel und Trägerstoffe in eine Form gebracht, die die unmittelbare oder mittelbare lokale Applikation im Wundgebiet gestattet.

Die Aufgabe wurde dadurch gelöst, daß das Peptid Lys-Pro, eine Teilsequenz u. a. des physiologischen Regulatorpeptids Substanz P (OEHME, P., M. BIENERT, K. HECHT und J. BERGMANN: Substanz P. In: OEHME, LÖWE, GÖRES: Beiträge zur Wirkstoffforschung, Heft 12, Akademie-Industrie-Komplex "Arzneimittelforschung", 1136 Berlin, Alfred-Kowalke-Str. 4), Lys-Pro-Derivate, wie Lys-Pro-Salze, geschützte Lys-Pro-Derivate sowie Peptide mit der Sequenz Lys-Pro - sowohl lineare als auch cyclische - und worin Prolin durch Pipecolinsäure, Hydroxyprolin, Dehydroprolin, $\beta$-Prolin, primäre und sekundäre heterocyclische Amine, insbesondere Pyrrolidin oder Piperidin, ersetzt sein kann, als Träger einer qualitativ neuen, nämlich die Bildung des Granulationsgewebes stimulierenden, Wirkung eingesetzt wird.

Eine bevorzugte Verbindung ist N-4-Nitrobenzylocarbonyl-lysyl-Prolin-Hydrochlorid. Seine Darstellung ist dadurch gekennzeichnet,
daß man ein Lysinderivat der Formel I

X-Lys [Z ($NO_2$)] - Y  I

mit einem Prolinderivat der Formel II

H-Pro-A  II,

worin

- X für tert.-Butyloxycarbonyl, p-Methoxybenzyloxycarbonyl, Biphenylisopropyloxycarbonyl, , -Dimethyl-3,5-dimethoxybenzyloxycarbonyl, o-Nitrophenylsulphenyl, 9-Fluorenylmethyloxycarbonyl
- Y für OH, Pentafluorphenyl, Pentachlorphenyl-, Trichlorphenyl-, p-Nitrophenyl-, N-Hydroxysuccinimidester, Hydrazid
- A für OH, Methyl-, Ethyl-, tert.-Butyl-, Phenyl-, Phenacylester

steht,
nach den in der Peptidchemie üblichen Kupplungsmethoden (vgl. E. WÜNSCH, Synthese von Peptiden im Houben-Weyl, Band 15/II, Methoden der organischen Chemie, Ed. E. MÜLLER, Georg Thieme Verlag Stuttgart 1974) N,N'-Dicyclohexylcarbodiimid, N,N'-Dicyclohexylcarbodiimid/Additiv (vorzugsweise 1-Hydroxy-benzotriazol), Mischanhydrid, aktivierte Ester, Azid-verknüpft und nach Reinigung des entsprechenden Dipeptidderivates durch Umkristallisieren oder durch Adsorbtionschromatographie an einer Kieselgelsäule die Amino- und Carboxylschutzgruppe mit den in der Peptidchemie üblichen Deblockierungsverfahren (vgl. E. WÜNSCH, s. o.), insbesondere mittels HCl/Dioxan, HCl/Eisessig, Trifluoressigsäure, Zink/Eisessig, Piperidin oder alkalische Hydrolyse entfernt, und das erhaltene Dipeptid H-Lys [Z($NO_2$)]-Pro-OH bevorzugt als Hydrochlorid isoliert. Vorzugsweise erfolgt die Peptidknüpfung über die Aktivestermethode durch Umsetzung von N-tert.-Butyloxycarbonyl-N-p-nitrobenzyloxycarbonyl-lysin-pentafluorphenylester mit Prolin in Gegenwart einer äquivalenten Menge Triethylamin in Dimethylformamid/Wasser (10 : 1). Der Reaktionsansatz wird 1 h bei 0 °C und 12 h bei Raumtemperatur gerührt, anschließend in eine $KHSO_4$-Lösung gegossen und das Rohprodukt mit Essigester extrahiert. Die vereinigten organischen Phasen werden mit $KHSO_4$-Lösung und Wasser gewaschen und über $Na_2SO_4$ getrocknet. Nach Verdampfen des Essigesters i. Vak. wird das dünnschichtchromatographisch einheitliche Produkt zur Entfernung der N-tert.-Butyloxycarbonyl-Schutzgruppe 40 Minuten mit 4,2 N HCl in Dioxan bei Raumtemperatur behandelt und das resultierende Dipeptid H-Lys [Z($NO_2$)]-Pro-OHals Hydrochlorid (Rf : 3,9 in n-Butanol/Eisessig/Wasser (4 : 1 : 1), Rf : 5,6 in n-Butanol/Eisessig/Wasser/Essigester (1 : 1 : 1 : 1), auf Kieselgel-Fertigplatten, Kavalier-

CSSR; $[D]^{22}$ = -35,8 [c = 1] Wasser) charakterisiert. Als bevorzugte Zubereitungen kommen Granulate, Lösungen, Emulsionen, Suspensionen und Aerosole in Betracht. Lösungen und Emulsionen können neben dem (oder den) Wirkstoff(en) die üblichen Trägerstoffe wie Lösungsmittel und Lösungsvermittler enthalten. Als Lösungsmittel können insbesondere Wasser oder niedere Alkohole sowie niedere Glykole wie Propylenglykol und 1,3-Butylenglykol verwendet werden. Als Lösungsvermittler können Verbindungen aus der Reihe der Polyglykole dienen, welche noch mit Ölen oder mit Glyzerin vermischt sein können.

Suspensionen können neben dem (oder den) Wirkstoff(en) die üblichen Trägerstoffe wie flüssige Verdünnungsmittel (z. B. Wasser, Ethanol, Propylenglykol) und Suspendiermittel enthalten, sofern diese wundverträglich sind.

Erfindungsgemäß wird der Wirkstoff als Zusatz zu Kosmetika verwendet. In Rasierwasser für die Hautbehandlung nach der Naßrasur dient er der Abheilung gesetzter Schnittverletzungen oder bestehender andersartiger Hautwunden. In Form eines Kosmetikstiftes ermöglicht er die gezielte Behandlung von kleinflächigen Hautwunden unterschiedlicher Genese. Als Bestandteil von Zahnpasten übt er einen heilenden Effekt auf bestehende Zahnfleischwunden aus. Als Zusatz zu Mund- und Gurgelwasser sind durch ihn entzündlich-geschwürige Schleimhautdefekte im Mund- und Rachenraum, beispielsweise im Verlauf von Viruskrankheiten, günstig zu beeinflussen. Das gilt gleichermaßen für die lokale Behandlung solcher Veränderungen beim Tier, z. B. im Verlauf von Virusexanthemen oder bestimmten Stoffwechselstörungsformen.

Ein weiteres Indikationsgebiet besteht in der Behandlung und Konditionierung großflächiger Hautwunden, z. B. operativ gesetzter Wunden, zur Vorbereitung einer plastischen Deckung, beim Ulcus cruris oder von Brandwunden. Hier wird nach Säuberung des Wundgrundes die Bildung von Granulationsgewebe dadurch unterstützt und gezielt gefördert, daß Wundabdeckungsmaterial, beispielsweise Kunststoffweichschaumband wie SYSpur-derm, mit dem Wirkstoff imprägniert oder physikalisch wirkende Granulate wie das compoundierte Zelluloseregenerat Deshisan mit dem Wirkstoff versetzt werden.

Erfindungsgemäß enthält die Zubereitung den die Granulationsgewebsbildung stimulierenden Wirkstoff in Konzentrationen zwischen 10 und 100 µg pro ml bzw. g.

Die Bedeutung der Erfindung ergibt sich daraus, daß mittels des erfindungsgemäßen Wirkstoffes allein oder seiner wahlweisen Kombination mit einem anderen gebräuchlichen Wirkstoff mit differentem Angriffspunkt und mit nichttoxischen, inerten, pharmazeutisch geeigneten Trägerstoffen Zubereitungen hergestellt werden können, die verwendbar sind sowohl in der Medizin zur Förderung der Heilung von Wunden, insbesondere solchen mit starken Gewebsdefekten, bei denen die Auslösung, Anregung und Verstärkung der Granulationsgewebsbildung für den Heilungsvorgang von ausschlaggebender Bedeutung sind und wahlweise gleichzeitig hemmende Einflüsse auf unliebsame Begleitprozesse wie Eiterung ausgeübt werden sollen als auch in der Kosmetik zur Verbesserung der Wirkungsqualität und -quantität einschlägiger Fabrikate für die Haut- und Zahn- bzw. Zahnfleischpflege.

Die Erfindung soll anhand von Ausführungsbeispielen näher erläutert werden.

## Ausführungsbeispiele

Abkürzungsverzeichnis

| | |
|---|---|
| FG | Feuchtgewicht |
| FG % | Prozentuale Veränderung des Feuchtgewichtes |
| TG | Trockengewicht |
| TG % | Prozentuale Veränderung des Trockengewichtes |
| TZ | Tierzahl |
| x | Die Steigerungsraten der Feucht- und Trockengewichte können im t-Test nach STUDENT signifikant gesichert werden. |

### Beispiel 1:

Anwendung des Wirkstoffes Lys $[Z(NO_2)]$-Pro•HCl in wäßriger Lösung am Rattenwundheilungsmodell nach RUDAS.

Unter die Rückenhaut von 2 Monate alten Ratten mit einem Körpergewicht von 220 bis 270 g werden Polyvinylringe mit einem Basisdurchmesser von 19 mm unter sterilen Kautelen implantiert (Test nach RUDAS, modifiziert nach BECK, WIEGERS-HAUSEN und JUNG, Acta bio. med. Germ. 13, (1964)).

Der Ring liegt auf der Fascis der Rückenmuskulatur. Am Operationstag werden je 1,0 ml sterile, isotone

Kochsalzlösung (Kontrolle) bzw. die gleiche Menge an Kochsalzlösung mit den darin enthaltenen Wirkstoffen appliziert. Die Wunde der Rückenhaut über dem Ring wird danach verschlossen. Am 2. bzw. am 2., 4. und 6. Tag post operationem (p. op.) erfolgen nochmalige Injektionen durch die Haut in den Ring, wobei jeweils 0,2 ml sterile, isotone Kochsalzlösung mit jeweils 20 % der am Operationstag gegebenen Wirkstoffmenge appliziert werden. Im Fall der Kontrolle erfolgt eine Injektion von 0,2 ml Kochsalzlösung ohne Wirkstoff.
3 und 7 Tage post operationem erfolgt die quantitative Gewinnung des im Ring neugebildeten Granulationsgewebes (Wundgewebe) durch Abpräparation von der Faszie. Danach wird das Feuchtgewicht des Gewebes mittels Torsionswaage bestimmt. Die Trockengewichte werden nach Extraktion des Wassers durch zweimalige 24stündige Lagerung in Ether und 48stündige Trocknung bei 56 °C ermittelt (NEUMANN und LOGAN, J. Biol. Chem. 184, 299 (1950)).

Beispiel 2:

Anwendung des Wirkstoffes zur Imprägnation von synthetischem Hautersatz.

Aus dem synthetischen Hautersatz "SYSpur-derm" (Hersteller: VEB Synthesewerk Schwarzheide/DDR) werden mit einem scharfen Locheisen Scheiben von 13 mm Durchmesser unter sterilen Bedingungen ausgestanzt. Diese Scheiben werden entweder in sterile, isotone Kochsalzlösung (0,14 M) (Kontrolle) oder in steriler, isotoner Kochsalzlösung, der 50 µg Lys-Pro/ml zugesetzt werden, eingelegt. Die Flüssigkeitsaufnahme pro Scheibe beträgt etwa 0,07 ml und der Lys-Pro-Gehalt demzufolge etwa 5 µg.
Unter die Rückenhaut von etwa 2 Monate alten, männlichen Ratten mit einem Körpergewicht von 230 bis 270 g werden wiederum Polyvinylringe (Test nach RUDAS, vgl. Beispiel 1) unter sterilen Kautelen implantiert. Die wirkstofffreien oder die mit Wirkstoff imprägnierten "SYSpur-derm"-Scheiben werden während der

Wirkung unterschiedlicher Dosen von Lys /¯Z($NO_2$)_7-Pro·HCl pro ml isotoner Kochsalzlösung

Tabelle 1: Feucht-(FG) und Trockengewichte (TG) sowie Wassergehalt von Granulationsgewebe 2 Monate alter Ratten 3 Tage post operationem

| | Kontrolle | 5 µg/ml | 50 µg/ml | 100 µg/ml | 500 µg/ml |
|---|---|---|---|---|---|
| FG /mg/ | 125,2 ± 5,6 | 125,0 ± 7,8 | 231,4 ± 3,2 | 188,6 ± 34,2 | 209,6 ± 14,2 |
| TG /mg/ | 11,9 ± 0,5 | 12,6 ± 0,8 | 24,5 ± 1,6 | 18,8 ± 3,4 | 21,3 ± 2,1 |
| $H_2O$ % | 90,4 ± 0,3 | 89,8 ± 0,6 | 89,4 ± 0,7 | 89,8 ± 0,4 | 89,8 ± 0,7 |
| TZ | 21 | 6 | 7 | 7 | 7 |
| FG % | 100 | 99,8 | 184,8 | 150,6 | 167,4 |
| TG % | 100 | 105,9 | 205,9 | 158,0 | 179,0 |

Wirkung unterschiedlicher Dosen von Lys/¯Z($NO_2$)_7-Pro·HCl pro ml isotoner Kochsalzlösung

Tabelle 2: Feucht- und Trockengewichte sowie Wassergehalt von Granulationsgewebe 2 Monate alter Ratten 7 Tage post operationem

| | Kontrolle | 5 µm/ml | 50 µg/ml | 100 µg/ml | 500 µg/ml |
|---|---|---|---|---|---|
| FG /mg/ | 162,2 ± 9,9 | 260,7 ± 21,2 | 235,0 ± 9,7 | 242,8 ± 20,2 | 223,6 ± 5,0 |
| TG /mg/ | 14,7 ± 1,1 | 27,4 ± 2,3 | 26,6 ± 1,6 | 24,9 ± 2,2 | 26,3 ± 0,6 |
| $H_2O$ % | 90,9 ± 0,3 | 89,4 ± 0,4 | 88,7 ± 0,3 | 89,4 ± 0,4 | 88,2 ± 0,3 |
| TZ | 9 | 7 | 7 | 7 | 7 |
| FG % | 100 | 160,7 | 144,9 | 149,7 | 137.9 |
| TG % | 100 | 186,4 | 181,0 | 169,4 | 179,0 |

Operation innerhalb der Ringe auf die Faszie der Rückenmuskulatur aufgelegt. Sie bedecken den größten Teil der Wunde und haften auf der Faszie. Die Wunde über dem Ring wird sodann mit Hautklammern verschlossen. 3 und 7 Tage Post operationem (p. op.) erfolgt die quantitative Gewinnung des im Ring neugebildeten Granulationsgewebes bzw. Wundgewebes (vgl. Beispiel 1). Dazu wird zunächst jede "SYSpur-derm"-Scheibe vom Granulationsgewebe abgehoben und das Granulationsgewebe selbst von der

Faszie abpräpariert. Die weitere Bearbeitung erfolgt in der unter Beispiel 1 beschriebenen Art und Weise. Folgende Versuchsgruppen wurden gebildet:

| | | |
|---|---|---|
| Gruppe 1 | 3 Tage p. op. | SYSpur-derm-Scheiben-Kontrolle (mit steriler isotoner Kochsalzlösung) |
| Gruppe 2 | 3 Tage p. op. | Imprägnierte "SYSpur-derm"-Scheiben (wie oben beschrieben) |
| Gruppe 3 | 7 Tage p. op. | SYSpur-derm-Scheiben-Kontrolle (mit steriler isotoner Kochsalzlösung) |
| Gruppe 4 | 7 Tage p. op. | Imprägnierte "SYSpur-derm"-Scheiben (wie oben beschrieben) |

Wundheilungsstörungen traten nicht häufig auf. Bei drei Tieren bestand eine Infektion infolge Aufbeißen des Wundverschlusses. Diese Tiere wurden aus dem Versuch ausgesondert. 7 Tage p. op. waren die "SYSpur-derm"-Scheiben fest mit dem neugebildeten Granulationsgewebe verwachsen, und bei ihrem Abheben traten nach 7 Tagen stärker als nach 3 Tagen p. op. punktförmige Blutungen der Wunde auf.

Tabelle 3

| Untersuchungsergebnisse 3 Tage p. op. | | | |
|---|---|---|---|
| | Gruppe 1 Kontrolle | Gruppe 2 imprägniert | Steigerung % |
| Feuchtgewichte (mg) | 138 ± 15,9 | 187 ± 13,9 | 35,5 $^{+)}$ |
| Trockengewichte (mg) | 13,5 ± 1,7 | 17,6 ± 1,0 | 30,4 $^{+)}$ |
| Tierzahl | 9 | 10 | |

Tabelle 4

| Untersuchungsergebnisse 7 Tage p. op. | | | |
|---|---|---|---|
| | Gruppe 3 Kontrolle | Gruppe 4 imprägniert | Steigerung % |
| Feuchtgewichte (mg) | 140 ± 10,7 | 169 ± 7,2 | 21,7 $^{+)}$ |
| Trockengewichte (mg) | 16,2 ± 1,1 | 18,6 ± 0,6 | 14,8 |
| Tierzahl | 11 | 13 | |

Die Steigerungsraten der Feucht- und Trockengewichte konnten im t-Test nach STUDENT signifikant gesichert werden (+).

Die im Vergleich zu den Ergebnissen nach wiederholter Gabe von Wirkstofflösung (siehe Beispiel 1) geringeren Steigerungsraten, insbesondere bei Ratten 7 Tage p. op., sind durch kleinere Mengen des zugefügten Wirkstoffes pro Scheibe (unter 5 µg) zu erklären.

**Beispiel 3:** Testung von Lys-Pro·2HBr

Das Vorgehen entspricht dem unter Beispiel 1.

**Tabelle 5:** Wirkung unterschiedlicher Dosen von Lys-Pro·2HBr pro ml isotoner Lösung. Feucht- und Trockengewichte sowie Wassergehalt von Granulationsgewebe 2 Monate alter Ratten 3 Tage post operationem

| | Kontrolle | 50 µg/ml | 500 µg/ml |
|---|---|---|---|
| FG | 125,2 ± 5,6 | 170,7 ± 8,7 +) | 193,6 ± 20,3 +) |
| TG | 11,9 ± 0,5 | 16,6 ± 1,5 +) | 18,7 ± 2,0 +) |
| $H_2O$ | 90,4 ± 0,3 | 90,3 ± 0,7 | 90,3 ± 0,2 |
| TZ | 21 | 7 | 7 |
| FG % | 100 | 136,3 | 154,6 |
| TG % | 100 | 139,5 | 157,1 |

**Tabelle 6:** Wirkung unterschiedlicher Dosen von Lys-Pro·2HBr pro ml isotoner Lösung. Feucht- und Trockengewichte sowie Wassergehalt von Granulationsgewebe 2 Monate alter Ratten 7 Tage post operationem

| | Kontrolle | 50 µg/ml | 500 µl/ml |
|---|---|---|---|
| FG | 132,5 ± 6,9 | 184,2 ± 13,5 +) | 186,0 ± 10,4 +) |
| TG | 13,6 ± 1,1 | 25,4 ± 1,9 +) | 25,4 ± 2,8 +) |
| $H_2O$ | 89,6 ± 0,7 | 85,7 ± 1,7 | 85,9 ± 2,1 |
| TZ | 10 | 6 | 6 |
| FG % | 100 | 139,0 | 140,4 |
| TG % | 100 | 186,8 | 186,8 |

**Beispiel 4:** Testung von Lys(Z)-Pro

Das Vorgehen entspricht dem unter Beispiel 1.

**Tabelle 7:** Wirkung unterschiedlicher Dosen von Lys(Z)-Pro ml isotoner Kochsalzlösung. Feucht- und Trockengewichte sowie Wassergehalt von Granulationsgewebe 2 Monate alter Ratten 3 Tage post operationem

| 3d | Kontrolle | 5 µg/ml | 10 µg/ml | 20 µg/ml | 40 µg/ml |
|---|---|---|---|---|---|
| FG | 125,2 ± 5,6 | 121,0 ± 6,4 | 147,5 ± 14,5 | 206,7 ± 17,8 +) | 187,5 ± 15,3 +) |
| TG | 11,9 ± 0,5 | 9,4 ± 0,4 +) | 14,1 ± 2,3 | 16,5 ± 2,3 +) | 12,1 ± 1,1 |
| $H_2O$ | 90,4 ± 0,3 | 92,1 ± 0,4 +) | 90,6 ± 0,8 | 92,7 ± 1,8 | 93,5 ± 0,6 +) |
| TZ | 21 | 5 | 6 | 6 | 4 |
| FG % | 100 | 96,6 | 117,8 | 165,1 | 149,8 |
| TG % | 100 | 79,0 | 118,5 | 138,7 | 101,7 |

**Tabelle 8:** Wirkung unterschiedlicher Dosen von Lys(Z)-Pro ml isotoner Kochsalzlösung. Feucht- und Trockengewichte sowie Wassergehalt von Granulationsgewebe 2 Monate alter Ratten 7 Tage post operationem

| | Kontrolle | | | | |
|---|---|---|---|---|---|
| FG | 162,2 ± 9,9 | 121,0 ± 11,1 +) | 119,5 ± 13,0 +) | 114,0 ± 11,2 +) | 125 ± 12,0 +) |
| TG | 14,7 ± 1,1 | 13,1 ± 1,1 | 11,8 ± 0,8 | 12,6 ± 1,2 | 13,1 ± 1,5 |
| $H_2O$ | 90,9 ± 0,3 | 89,1 ± 0,4 +) | 90.0 ± 0,6 | 88,9 ± 0,3 +) | 89.6 ± 0,4 +) |
| TZ | 9 | 5 | 6 | 5 | 6 |
| FG % | 100 | 74,6 | 73,7 | 70,3 | 77,1 |
| TG % | 100 | 89,1 | 80,3 | 85,7 | 89,1 |

Beispiel 5: Testung des Tetrapeptids SP $^{(1-4)}$

Das Vorgehen entspricht dem unter Beispiel 1.

Tabelle 9: Wirkung unterschiedlicher Dosen von SP $^{(1-4)}$ pro ml isotoner Kochsalzlösung. Feucht- und Trockengewichte sowie Wassergehalt von Granulationsgewebe 2 Monate alter Ratten 3 Tage post operationem

| 3d | Kontrolle SP $^{(1-4)}$ | 5 µg/ml | 10 µg/ml | 50 µg/ml | 100 µg/ml |
|---|---|---|---|---|---|
| FG | 125,2 ± 5,6 | 200,7 ± 24,9 +) | 344,0 ± 57,9 +) | 342,9 ± 27,1 +) | 371,4 ± 30,4 +) |
| TG | 11,9 ± 0,5 | 21,5 ± 2,6 +) | 43,2 ± 2,1 +) | 37.9 ± 3,5 +) | 39,9 ± 2,2 +) |
| $H_2O$ | 90,4 ± 0,3 | 89,2 ± 0,3 +) | 88,9 ± 0,4 +) | 89,0 ± 0,4 +) | 89,1 ± 0,4 +) |
| TZ | 21 | 7 | 7 | 7 | 7 |
| FG % | 100 | 160,3 | 274,8 | 273,9 | 296,6 |
| TG % | 100 | 180,7 | 363,0 | 318,5 | 335,3 |

Tabelle 10: Wirkung unterschiedlicher Dosen von SP $^{(1-4)}$ pro ml isotoner Kochsalzlösung. Feucht- und Trockengewichte sowie Wassergehalt von Granulationsgewebe 2 Monate alter Ratten 7 tage post operationem

| | Kontrolle | | | |
|---|---|---|---|---|
| FG | 162,2 ± 9,9 | 209,2 ± 14,5 +) | 290,7 ± 24,9 +) | 256.7 ± 21,2 +) |
| TG | 14,7 ± 1,1 | 24,0 ± 1,8 +) | 33,3 ± 2,9 | 31,6 ± 2,9 +) |
| $H_2O$ | 90,9 ± 0,3 | 88,3 ± 1,2 | 88,3 ± 0,5 +) | 87,6 ± 0,7 +) |
| TZ | 9 | 6 | 7 | 6 |
| FG % | 100 | 128,9 | 179,2 | 158,3 |
| TG % | 100 | 163,3 | 226.5 | 215.0 |

Beispiel 6:

Histologisch-morphometrische Untersuchungen zum Nachweis der Angiogenese fördernden Wirkung von Lys[Z($NO_2$)]-Pro•HCl

An histologischen Schnitten wurde eine Differentialzählung der Gesamtzellzahl ($N_{NVC}$) unterteilt nach der Anzahl der Zellkerne der Fibroblasten ($N_{NCTC}$), Granulozyten ($N_{NGC}$), der Rundzellen ($N_{NRC}$) und Endothelzellen ($N_{NEC}$) bei einer 100fachen Vergrößerung in Ölimmersion und mit einem Okularnetzmikrometer durchgeführt (P. BUNTROCK: Zbl. allg. Pathol. u. allg. pathol. Anat., 124 (1980) 48-59). In der Tabelle 11 sind die Ergebnisse dargestellt. Als Kontrolltiere dienten mit isotoner Kochsalzlösung behandelte Ratten, deren Zählwerte gleich 100 % gesetzt wurden. Daraus ergibt sich eine signifikante Zunahme der Anzahl der Endothelzellkerne der Kapillaren. Das Vorgehen der Behandlung entspricht dem Beispiel 1.

Tabelle 11: Prozentuale Veränderung der Anzahl der Zellkerne Kontrollen wurden gleich 100 % gesetzt.

| Wundalter: | 3 Tage p. op. | | 7 Tage p. op. | |
|---|---|---|---|---|
| Behandlung: | Wiederholte Gabe von Lys[Z($NO_2$)]-Pro•HCl | | | |
| | 50 µg/ml | 500 µg/ml | 50 µg/ml | 500 µg/ml |
| $N_{NVC}$ | 96 | 108 | 95 | 85 |
| $N_{NOTC}$ | 95 | 117 | 71 | 66 |
| $N_{NGC}$ | 99 | 24 | 85 | 86 |
| $N_{NRC}$ | 61 | 67 | 90 | 78 |
| $N_{NEC}$ | 230 | 292 | 174 | 148 |

Mit Hilfe des Punktzählverfahrens (BUNTROCK et al: Exper. Pathol. 21 (1982) 62-67) wurden die prozentualen Volumenanteile der Kapillaren ($VV_{CAP}$), unterteilt nach Endothelzellen ($VV_{EC}$), Endothelzellkerne ($VV_{NEC}$), Endothelsellzytoplasma ($VV_{CYT}$) einerseits und Kapillarlumen ($VV_{CAPL}$) andererseits bestimmt. Die Kontrollen wurden wiederum mit 100 % festgelegt.
Die Ergebnisse sind in der Tabelle 12 dargestellt.

Tabelle 12: Morphometrie der Kapillaren

| Wundalter: | 3 Tage p. op. | | 7 Tage p. op. | |
|---|---|---|---|---|
| Behandlung: | Wiederholte Gabe von Lys[Z($NO_2$)]-Pro•HCl | | | |
| | 50 µg/ml | 500 µg/ml | 50 µg/ml | 500 µg/ml |
| $VV_{CAF}$ | 140 | 157 | 200 | 300 |
| $VV_{EC}$ | 155 | 185 | 192 | 266 |
| $VV_{NEC}$ | 214 | 274 | 260 | 409 |
| $VV_{CYT}$ | 127 | 142 | 157 | 193 |
| $VV_{CAPL}$ | 109 | 114 | 230 | 374 |

Dadurch kann die Angiogenese fördernde Wirkung durch das Dipeptid Lys[Z($NO_2$)]-Pro•HCl als Ursache für die vermehrte Bildung von Granulationsgewebe und für die beschleunigte Wundheilung gesehen werden.

Beispiel 7:

Wundheilungsförderndes Rasierwasser

Durch Vermischen von Ethanol, Adstringentien, bakteriostatisch wirksamen Substanzen, Duftstoffen werden durch Zusatz von 0,0001 bis 0,1 % Lys-Pro bzw. seinen Derivaten wundheilungsfördernde Rasierwässer hergestellt.

Beispiel 8:

Wundheilungsfördernde Zahnpasten

Durch Vermischen von Putzkörpern, Schaummitteln, Feuchthaltemitteln, Aromastoffen, Konservierungsmitteln, Verdickungsmitteln und unter Zusatz von 0,0001 bis 0,1 % Lys-Pro bzw. seinen Derivaten werden wundheilungsfördernde Zahnpasten hergestellt.

Beispiel 9:

Wundheilungsfördernde Mund- und/oder Gurgelwässer

Durch Vermischen von Ethanol, Aromastoffen und unter Zusatz von 0,0001 bis 0,1 % Lys-Pro bzw. seinen Derivaten werden Mund- und/oder Gurgelwässer hergestellt, die die Ausheilung von Schleimhautwunden im Mund- und Rachenraum fördern.

Beispiel 10:

Wundbehandlungsstifte

Durch Vermischen von Lippenstiftgrundlagen und 0,0001 bis 0,1 % Lys-Pro bzw. seinen Derivaten werden Wundbehandlungsstifte hergestellt.

Beispiel 11:

Wundheilungsförderndes Verbandsmaterial

Verbandsmaterial, vorzugsweise Kunststoffweichschaumband, wird entweder bei der industriellen Fertigung, beispielsweise über ein System parallel angeordneter Spritzdüsen, bevorzugt mit einer $5 \times 10^{-3}$ bis $5 \times 10^{-5}$ molaren, sterilen, wäßrigen Lys-Pro- bzw. Lys-Pro-Derivat-Lösung streifenweise getränkt, getrocknet und steril verpackt oder im Klinikum unmittelbar vor der Anwendung in eine ebenso konzentrierte keimfreie Lys-Pro- bzw. Lys-Pro-Derivat-Lösung eingetaucht und unmittelbar auf die Wundfläche gelegt.

Beispiel 12:

Wundheilungsförderndes Wundpulver

Adsorbierendes Wundpulver, vorzugsweise compoundiertes Zelluloseregenerat, wird mit Lys-Pro- bzw. Lys-Pro-Derivat-Trockensubstanz im bevorzugten Verhältnis 1 : 100 bis 1 : 10 000 versetzt und vermischt.

**Patentansprüche**
**Patentansprüche für folgende Vertragsstaaten : AT, BE, CH, DE, FR, GB, IT, LI, SE**

1. Verwendung eines Peptids Lys-Pro oder von einem Derivat desselben zur Herstellung einer Präparation, die die Wundheilung fördert.

2. Verwendung nach Anspruch 1, dadurch gekennzeichnet, daß man das Peptid Lys-Pro oder ein Derivat davon in einer Menge von 1 µg bis 1 mg pro ml bzw. g der fertigen Präparation einsetzt.

3. Verwendung nach Anspruch 1 oder 2, dadurch gekennzeichnet, daß man Salze, geschützte Lys-Pro-Derivate und Peptide mit der Sequenz Lys-Pro, sowohl lineare als auch cyclische, einsetzt, und worin Prolin durch Picolinsäure, Hydroxyprolin, Dehydroprolin, β-Prolin, primäre und sekundäre heterocyclische Amine, insbesondere Pyrrolidin order Piperidin, ersetzt sein kann.

**4.** Verwendung nach Anspruch 2 oder 3 zur Herstellung von wundheilungsfördernden Rasierwässern auf der Basis von Ethanol, Adstringentien, bakteriostatisch wirkenden Substanzen, und Duftstoffen.

**5.** Verwendung nach Anspruch 2 oder 3 zur Herstellung von Zahnpasten, die einen heilenden Effekt auf Zahnfleischwunden ausüben, auf der Basis von Putzkörpern, Schaummitteln, Feuchthaltemitteln, Aromastoffen, Konservierungsmitteln, Verdickungsmitteln.

**6.** Verwendung nach Anspruch 2 oder 3 zur Herstellung von Mund- und/oder Gurgelwässern, die die Ausheilung von Schleimhautwunden im Mund- und Rachenraum fördern, auf der Basis von Ethanol und Aromastoffen.

**7.** Verwendung nach Anspruch 2 oder 3 zur Herstellung eines Wundbehandlungsstiftes auf der Basis von Lippenstiftgrundlage.

**8.** Verwendung nach einem der Ansprüche 1 bis 3 zur Herstellung von Verbandmaterial, vorzugsweise auf der Basis eines Kunststoffweichschaumbands, durch streifenweises Tränken mit einer vorzugsweise 5 x $10^{-3}$ bis 5 x $10^{-5}$ molaren, sterilen, wässerigen Lys-Pro- bzw. Lys-Pro-Derivat-Lösung.

**9.** Verwendung nach einem der Ansprüche 1 bis 3 zur Herstellung eines wundheilungsfördernden Wundpulvers durch Vermischen von adsorbierendem Wundpulver, vorzugsweise compoundiertem Zelluloseregenerat, mit Lys-Pro- bzw. Lys-Pro-Derivat-Trockensubstanz, vorzugsweise im Verhältnis von 1 : 100 bis 1 : 10000.

**10.** Verwendung nach einem der Ansprüche 1 bis 9, dadurch gekennzeichnet, daß man N-4-Nitrobenzyloxycarbonyl-Lysyl-Prolin-Hydrochlorid(H-Lys[Z($NO_2$)]-Pro-OH.HCl) einsetzt.

**Patentansprüche für folgenden Vertragsstaat : AT**

**1.** Verfahren zur Herstellung einer Präparation, die die Wundheilung fördert, dadurch **gekennzeichnet,** dass man ein Peptid Lys-Pro oder ein Derivat desselben mit einem üblichen Trägerstoff formuliert.

**2.** Verfahren nach Anspruch 1, dadurch **gekennzeichnet,** dass man das Peptid Lys-Pro oder ein Derivat davon in einer Menge von 1 µg bis 1 mg pro ml bzw. g der fertigen Präparation einsetzt.

**3.** Verfahren nach Anspruch 1 oder 2, dadurch **gekennzeichnet,** dass man Salze, geschützte Lys-Pro-Derivate und Peptide mit der Sequenz Lys-Pro, sowohl lineare als auch cyclische, einsetzt, und worin Prolin durch Picolinsäure, Hydroxyprolin, Dehydroprolin, β-Prolin, primäre und sekundäre heterocyclische Amine, insbesondere Pyrrolidin oder Piperidin, ersetzt sein kann.

**4.** Verfahren nach Anspruch 2 oder 3, dadurch **gekennzeichnet,** dass man wundheilungsfördernden Rasierwässern Ethanol, Adstringentien, bakteriostatisch wirkende Substanzen oder Duftstoffe zufügt.

**5.** Verfahren nach Anspruch 2 oder 3, dadurch **gekennzeichnet,** dass man Zahnpasten, die einen heilenden Effekt auf Zahnfleischwunden ausüben, Putzkörper, Schaummittel, Feuchthaltemittel, Aromastoffe, Konservierungsmittel oder Verdickungsmittel zufügt.

**6.** Verfahren nach Anspruch 2 oder 3, dadurch **gekennzeichnet,** dass man Mund- und/oder Gurgelwässern, die die Ausheilung von Schleimhautwunden im Mund- und Rachenraum fördern, Ethanol oder Aromastoffe zufügt.

**7.** Verfahren nach Anspruch 2 oder 3, dadurch **gekennzeichnet,** dass man einen Wundbehandlungsstift auf der Basis einer Lippenstiftgrundlage formuliert.

**8.** Verfahren nach einem der Ansprüche 1 bis 3, dadurch **gekennzeichnet,** dass man Verbundmaterial, vorzugsweise auf der Basis eines Kunststoffweichschaumbandes, durch streifenweises Tränken mit einer vorzugsweise 5 x $10^{-3}$ - 5 x $10^{-5}$ molaren, sterilen, wässrigen Lys-Pro- bzw. Lys-Pro-Derivat-Lösung herstellt.

9. Verfahren nach einem der Ansprüche 1 bis 3, dadurch **gekennzeichnet,** dass man ein wundheilungsförderndes Wundpulver durch Vermischen von adsorbierendem Wundpulver, vorzugsweise compoundiertem Zelluloseregenerat, mit Lys-Pro- bzw. Ly-Pro-Derivat-Trockensubstanz, vorzugsweise im Verhältnis von 1:100 bis 1:10,000 formuliert.

10. Verfahren nach einem der Ansprüche 1 bis 9, dadurch **gekennzeichnet,** dass man als Wirkstoff N-4-Nitrobenzyloxycarbonyl-Lysyl-Prolin-Hydrochlorid(H-Lys[Z($NO_2$)]-Pro-OH.HCl) einsetzt.

11. Verwendung eines Peptids Lys-Pro oder eines Derivates desselben, wie es nach einem oder mehreren der Verfahren gemäss Ansprüchen 1 bis 10 erhalten wird, zur Herstellung einer Präparation, die die Wundheilung fördert.

**Claims**
**Claims for the following Contracting States : AT, BE, CH, DE, FR, GB, IT, LI, SE**

1. Use of a peptide Lys-Pro or a derivative thereof for producing a preparation which promotes wound healing.

2. Use according to claim 1, characterised in that the peptide Lys-Pro or a derivative thereof is used in an amount of 1 $\mu$g to 1 mg per ml or g of the final preparation.

3. Use according to claim 1 or 2, characterised in that salts, protected Lys-Pro derivatives and peptides having the sequence Lys-Pro, both linear and cyclic, are used, and wherein proline may be replaced by picolinic acid, hydroxyproline, dehydroproline, $\beta$-proline, primary and secondary heterocyclic amines, in particular pyrrolidine or piperidine.

4. Use according to claim 2 or 3 for producing shaving lotions which promote wound healing and are based on ethanol, astringents, bacteriostatically active substances and fragrances.

5. Use according to claim 2 or 3 for producing toothpastes which have a healing effect on gum wounds and are based on cleaning bodies, foaming agents, humectants, aromatic substances, preservatives, thickeners.

6. Use according to claim 2 or 3 for producing mouthwashes and/or gargles which promote the healing of mucous membrane wounds in the mouth and pharynx and are based on ethanol or aromatic substances.

7. Use according to claim 2 or 3 for producing a wound treatment pencil based on a lipstick foundation.

8. Use according to one of claims 1 to 3 for producing dressing material, preferably based on a plastic soft foam tape, by strip-wise impregnation using a preferably 5 x $10^{-3}$ - 5 x $10^{-5}$ molar, sterile, aqueous Lys-Pro or Lys-Pro derivative solution.

9. Use according to one of claims 1 to 3 for producing a wound powder which promotes wound healing by mixing adsorbing wound powder, preferably compounded cellulose reclaim, with Lys-Pro or Lys-Pro derivative dry substance, preferably in the ratio of 1:100 to 1:10,000.

10. Use according to one of claims 1 to 9, characterised in that N-4-nitrobenzyloxycarbonyl-lysyl-proline hydrochloride (H-Lys-[Z($NO_2$)]-Pro-OH.HCl) is used.

**Claims for the following Contracting State : AT**

1. Process for producing a preparation which promotes wound healing, characterised in that a peptide Lys-Pro or a derivative thereof is formulated using a conventional excipient.

2. Process according to claim 1, characterised in that the peptide Lys-Pro or a derivative thereof is used

in an amount of 1 µg to 1 mg per ml or g of the final preparation.

3. Process according to claim 1 or 2, characterised in that salts, protected Lys-Pro derivatives and peptides having the sequence Lys-Pro, both linear and cyclic, are used, and wherein proline may be replaced by picolinic acid, hydroxyproline, dehydroproline, β-proline, primary and secondary heterocyclic amines, in particular pyrrolidine or piperidine.

4. Process according to claim 2 or 3, characterised in that ethanol, astringents, bacteriostatically active substances or fragrances are added to shaving lotions which promote wound healing.

5. Process according to claim 2 or 3, characterised in that cleaning bodies, foaming agents, humectants, aromatic substances, preservatives or thickeners are added to toothpastes which have a healing effect on gum wounds.

6. Process according to claim 2 or 3, characterised in that ethanol or aromatic substances are added to mouthwashes and/or gargles which promote healing of mucous membrane wounds in the mouth and pharynx.

7. Process according to claim 2 or 3, characterised in that a wound treatment pencil is formulated based on a lipstick foundation.

8. Process according to one of claims 1 to 3, characterised in that dressing material, preferably based on a plastic soft foam tape, is produced by strip-wise impregnation using a preferably $5 \times 10^{-3}$ - $5 \times 10^{-5}$ molar, sterile, aqueous Lys-Pro or Lys-Pro derivative solution.

9. Process according to one of claims 1 to 3, characterised in that a wound powder which promotes wound healing is formulated by mixing adsorbing wound powder, preferably compounded cellulose reclaim, with Lys-Pro or Lys-Pro derivative dry substance, preferably in the ratio of 1:100 to 1:10,000.

10. Process according to one of claims 1 to 9, characterised in that N-4-nitrobenzyloxycarbonyl-lysyl-proline hydrochloride (H-Lys-[Z($NO_2$)]-Pro-OH.HCl) is used as active ingredient.

11. Use of a peptide Lys-Pro or a derivative thereof, as obtained by one or more of the processes according to claims 1 to 10, for producing a preparation which promotes wound healing.

**Revendications**

**Revendications pour les Etats contractants suivants : AT, BE, CH, DE, FR, GB, IT, LI, SE**

1. Utilisation d'un peptide lys-pro ou l'un de ses dérivés pour la fabrication d'une préparation qui favorise la guérison des plaies.

2. Utilisation selon la revendication 1, caractérisé en ce que l'on emploie le peptide lys-pro ou l'un de ses dérivés en quantité de 1 µg à 1 mg par ml, ou par gramme de la préparation terminée.

3. Utilisation selon la revendication 1 ou 2 , caractérisée en ce que l'on emploie des sels, des dérivés de lys-pro protégés et des peptides de séquence lys-pro, tant linéaires que cycliques, et dans lesquels la proline peut être remplacée par l'acide picolinique, l'hydroxypyroline, la déhydroproline, la β-proline, des amines hétérocycliques primaires et secondaires, en particulier la pyrrolidine ou la pipéridine.

4. Utilisation selon la revendication 2 ou 3, pour la fabrication d'eaux de rasage à action cicatrisante, à base d'éthanol, d'astringents, de substances à action bactériostatique et de parfums.

5. Utilisation selon la revendication 2 ou 3, pour la fabrication de pâtes dentifrices exerçant un effet curatif sur les plaies gingivales, à base de corps nettoyants, d'agents moussants, d'humidificateurs, de substances aromatiques, d'agents de conservation, d'épaississants.

6. Utilisation selon la revendication 2 ou 3, pour la fabrication d'eaux de lavage de bouche et/ou de gargarisme, favorisant la guérison des plaies des muqueuses, dans la bouche et la cavité pharyngée, à

base d'éthanol et de substances aromatiques.

**7.** Utilisation selon la revendication 2 ou 3, pour la fabrication d'un bâtonnet de traitement des plaies à partir d'une matière de base de bâtonnet pour les lèvres.

**8.** Utilisation selon l'une des revendications 1 à 3, pour la fabrication d'un matériau de pansement, de préférence à base d'une bande de mousse souple en matière synthétique, par imbibation par bandes avec une solution stérile aqueuse de lys-pro, ou de dérivé de lys-pro, d'une concentration molaire allant de préférence de 5 x $10^{-3}$ à 5 x $10^{-5}$.

**9.** Utilisation selon l'une des revendications 1 à 3, pour la fabrication d'une poudre favorisant la guérison des plaies par mélange d'une poudre adsorbante pour plaies, de préférence constituée par un régénérat de cellulose compoundé, avec une quantité de matière sèche de lys-pro, ou d'un dérivé de lys-pro, correspondant de préférence à une proportion de 1:100 à 1:10 000.

**10.** Utilisation selon l'une des revendications 1 à 9, caractérisée en ce que l'on emploie le chlorhydrate de N-4-nitrobenzyloxycarbonyl-lysyl-proline (H-lys[Z($NO_2$)]-Pro-OH.HCl).

**Revendications pour l'Etat contractant suivant : AT**

**1.** Procédé de fabrication d'une préparation qui favorise la guérison des plaies, caractérisé en ce que l'on formule un peptide lys-pro ou l'un de ses dérivés avec un vecteur usuel.

**2.** Procédé selon la revendication 1, caractérisé en ce que l'on utilise le peptide lys-pro ou l'un de ses dérivés en quantité de 1 µg à 1 mg par ml, ou par gramme de la préparation terminée.

**3.** Procédé selon la revendication 1 ou 2, caractérisé en ce que l'on emploie des sels, des dérivés de lys-pro protégés et des peptides de séquence lys-pro, tant linéaires que cycliques, et dans lesquels la proline peut être remplacée par l'acide picolinique, l'hydroxyproline, la déhydroproline, la $\beta$-proline, des amines hétérocycliques primaires et secondaires, en particulier la pyrrolidine ou la pipéridine.

**4.** Procédé selon la revendication 2 ou 3, caractérisé en ce que l'on ajoute de l'éthanol, des astringents, des substances à action bactériostatique ou des substances aromatiques à des eaux de rasage favorisant la guérison des plaies.

**5.** Procédé selon la revendication 2 ou 3, caractérisé en ce que l'on ajoute des corps nettoyants, des agents moussants, des humidificateurs, des substances aromatiques, des agents de conservation ou des épaississants à des pâtes dentifrices exerçant un effet curatif sur les blessures gingivales.

**6.** Procédé selon la revendication 2 ou 3, caractérisé en ce que l'on ajoute de l'éthanol ou des substances aromatiques à des eaux de lavage de bouche et/ou de gargarisme, qui favorisent la guérison de plaies des muqueuses, dans la bouche et la cavité pharyngée.

**7.** Procédé selon la revendication 2 ou 3, caractérisé en ce que l'on formule un bâtonnet de traitement des blessures à partir d'une matière de bâtonnet pour les lèvres.

**8.** Procédé selon l'une des revendications 1 à 3, caractérisé en ce que l'on fabrique un matériau composite, de préférence à base d'une bande en mousse souple de matière synthétique, par imbibition par bandes avec une solution stérile aqueuse de lys-pro, ou de dérivé de lys-pro, d'une concentration molaire allant de préférence de 5 x $10^{-3}$ à 5 x $10^{-5}$.

**9.** Procédé selon l'une des revendications 1 à 3, caractérisé en ce que l'on formule une poudre favorisant la quérison des plaies par mélange de poudre adsorbante pour plaies, de préférence constituée par un régénérat de cellulose compoundé, avec une quantité de matière sèche de lys-pro ou d'un dérivé de lys-pro, correspondant de préférence à une proportion de 1:100 à 1:10.000.

**10.** Procédé selon l'une des revendications 1 à 9, caractérisé en ce que l'on utilise comme substance active le chlorhydrate de N-4-nitrobenzyloxycarbonyl-lysyl-proline (H-lys[Z($NO_2$)]-Pro-OH.HCl).

11. Utilisation d'un peptide lys-pro ou d'un de ses dérivés, tel qu'obtenu selon l'un ou plusieurs des procédés selon les revendications 1 à 10, pour la fabrication d'une préparation qui favorise la guérison des plaies.